# EUROPEAN PATENT SPECIFICATION

(11) **EP 1 392 276 B1**
(45) Date of publication and mention of the grant of the patent: **27.12.2006**
(21) Application number: 02724472.2
(22) Date of filing: 09.05.2002
(51) Int. Cl.: A61K 31/20, A61P 19/02, A61P 25/24, A61P 25/28, A61P 35/00, A61K 31/415, A61K 31/19, A61K 31/405

(54) **POTENTIATION OF THERAPEUTIC EFFECTS OF POLYUNSATURATED FATTY ACIDS**
VERSTÄRKTE WIRKUNG MEHRFACH UNGESÄTTIGTER FETTSÄUREN
POTENTIALISATION DES EFFETS THERAPEUTIQUES DES ACIDES GRAS

(30) Priority: 09.05.2001 GB 0111282
(43) Date of publication of application: 03.03.2004
(73) Proprietor: Amarin Neuroscience Limited, Stirling FK7 9JQ (GB)
(72) Inventor: HORROBIN, David Frederick, Kings Park House, Stirling FK7 9JQ (GB)
(74) Representative: Wakerley, Helen Rachael
(86) International application number: PCT/GB2002/002145
(87) International publication number: WO 2002/089787

(56) References cited:
- EP-A- 0 195 570
- EP-A- 0 675 103
- WO-A-01/60778
- US-A- 5 411 988
- ANDERSON K M ET AL: "Five-lipoxygenase inhibitors reduce PANC-1 survival: The mode of cell death and synergism MK886 with gamma linolenic acid." ANTICANCER RESEARCH, vol. 18, no. 2A, March 1998 (1998-03), pages 791-800, XP001097814 ISSN: 0250-7005

## Description

Unsaturated fatty acids of the omega-6 and omega-3 series have many potential uses. The present inventor and other inventors have obtained numerous patents and filed many patent applications which deal with the therapeutic effects of unsaturated fatty acids in many different disorders including cancers, skin disorders, inflammatory disorders, menstrual cycle disorders, reproductive disorders, renal and urinary tract disorders, metabolic disorders including diabetes mellitus, osteoporosis, urolithiasis and other disorders of calcium metabolism, gastrointestinal disorders, respiratory system disorders, and central nervous system disorders including neurological and psychiatric disorders. Examples of granted patents which demonstrate that these fatty acids have a wide range of utility in many diseases are the following US cases: US 4,826,877; 5,847,000; 5,457,130; 4,302,447; 4,681,896; 5,198,468; 5,922,345. WO 00/44361 discloses the use of omega-3 fatty acid, EPA, in the treatment of psychiatric or central nervous system disorders.

WO 01/60778 discloses the combination of EPA or AA with aspirin for the treatment of cancer, inflammatory and cardiovascular diseases.

This specification concerns methods for improving the efficacy of certain psychiatric and neurological or neurodegenerative disorder treatments with unsaturated fatty acids.

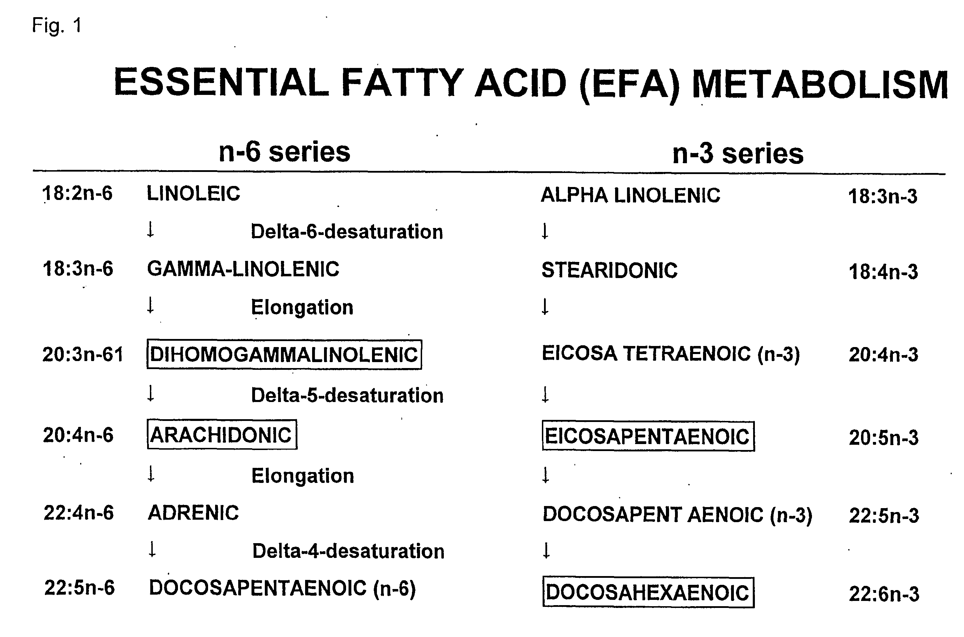

The pathways of metabolism of the unsaturated essential fatty acids (EFAs) are shown in figure 1. The EFAs are like vitamins in the sense that they are required for human and animal metabolism but cannot be synthesised de novo by the mammalian body. There are two sorts of EFAs: the n-6 (or omega-6) and the n-3 (or omega-3). The parent compounds linoleic acid (LA) of the n-6 series and alpha-linolenic acid (ALA) of the n-3 series are the main compounds found in the diet. However, to be useful to the body, these parent compounds must be converted to the so-called derived essential fatty acids shown in figure 1. These derived EFAs play key roles in the structures of all internal and external cell membranes. They are also released from these cell membranes following many different types of cell activation which convert phospholipases A₂, C and D to active forms and which directly or indirectly lead to release of the free acids from membrane phospholipids.

These free fatty acids then partake in many different signalling processes which modify many aspects of cellular function. The fatty acids which are of particular importance are three fatty acids which are good substrates for the cyclo-oxygenase(COX) group of enzymes, dihomogammalinolenic acid(DGLA),arachidonic acid (AA) and eicosapentaenoic acid (EPA) and another fatty acid, docosahexaenoic acid(DHA), which although a poor subject for COX is also an important component of membrane phospholipids. Gamma-linolenic acid(GLA), which is an effective precursor of DGLA and AA, and stearidonic acid(SA), which is an effective precursor of EPA, are also potentially important molecules.

There are two main types of COX. COX-1 is a constitutively expressed enzyme which continuously converts the relevant derived fatty acid to low to moderate levels of prostaglandins and related substances. COX-2 is an enzyme which is expressed in large amounts in most tissues when they are reacting to any form of change or stimulation. Thus COX-2 is expressed in large amounts whenever there is an inflammatory process of any sort, whenever cells proliferate abnormally as in cancer cells and the blood vessels supplying them, and in any situation where cells are dying or degenerating including neurodegenerative disorders like Parkinson's disease Alzheimer's disease,other forms of dementia,including vascular dementia, amyotrophic lateral sclerosis, Huntington's disease and other neurological disorders involving "triplet repeats" such as Friedreich's ataxia, spinocerebellar ataxia and myotonic dystrophy.

The free fatty acids released by phospholipases can also be converted to a range of other eicosanoids by a group of enzymes known as lipoxygenases (LOX). The products of the COX and LOX enzymes are believed to mediate many and perhaps most of the biological actions of the free fatty acids. The other major routes of disposal of the free fatty acids are oxidation and linkage to coenzyme A by a group of enzymes known as fatty acid coenzyme-A ligase (FACL) or alternatively as acyl-CoA synthetase (ACS). Linkage to CoA is a necessary step prior to the entry of fatty acids into any one of a large number of synthetic and degradative pathways.

There have been many proposals concerning the therapeutic uses of EFAs and derived EFAs, particularly GLA, DGLA and to some extent AA of the n-6 series, and EPA, docosapentaenoic acid (DPA), DHA and to some extent SA of the n-3 series. Most of these proposals have assumed that a substantial part of the therapeutic effects of the EFAs and derived EFAs depend on their conversion to highly active metabolites by the COX and LOX groups of enzymes.

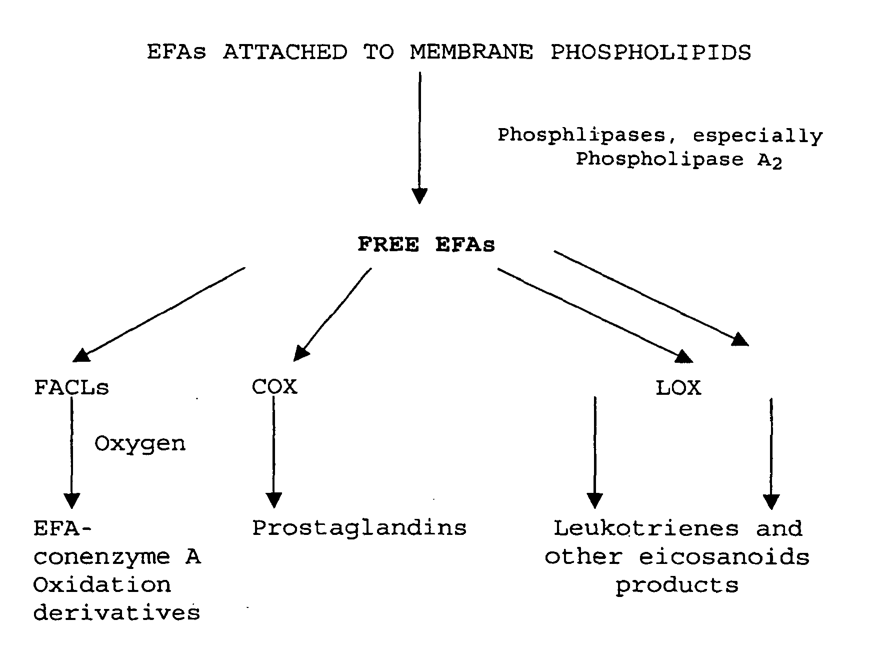

However, there is increasing evidence that many of the actions of the EFAs and derived EFAs are mediated not by the metabolites but by the fatty acids themselves. There appear to be many different mechanisms. As examples, some ion channels have binding sites for EFAs and their function can be modified, so regulating the movements of sodium, potassium, calcium and chloride channels. Or some protein kinases and other enzymes have allosteric binding sites for fatty acids which lead to their activation or inhibition. Or some genes may be directly regulated by the binding of fatty acids to DNA. Or some receptors, notably the various types of peroxisome proliferator activated receptors (PPAR) may be activated by fatty acids and lead to a wide range of changes in cellular function. Or some fatty acids may be able to cause cell death either by apoptosis (programmed cell death) or by other means. Interestingly some fatty acids, especially GLA, DGLA and EPA and to a lesser extent SA, AA and DHA seem to be able to kill malignant cells selectively without harming normal cells.

Fatty acids of different structures often interact with the same binding sites on enzymes, receptors, transport proteins and regulatory control sites. Different fatty acids may act as agonists, antagonists or have neutral effects at such sites. In the past it has been common for fatty acids with presumed therapeutic actions to be administered in the form of complex mixtures such as fish oils containing eicosapentaenoic acid and docosahexaenoic acid, or plant, algal, fungal or other microbial oils containing gamma-linolenic acid or arachidonic acid or stearidonic acid. These plant oils are often rich in linoleic acid. It has been assumed that the effect of the oil is that of the most biologically interesting fatty acid, though usually without any experimental evidence that this is the case. The present inventor has been investigating this and has found that the use of partially or fully purified fatty acids or fatty acid derivatives often produces therapeutic effects which are greater than expected on the basis of the known effects of the natural oils. Though not prior art, reference is made here to a paper by the present inventor:Horrobin, DF. A new category of psychotropic drugs: neuroactive lipids as exemplified by ethyl eicosapentaenoate. Progress in Drug Research, September 2002.

The present invention relates to specific therapeutic applications of formulations in which a particular EFA or derived EFA is used with an enzyme inhibitor selected from an inhibitor of COX-1 or COX-2 or LOX or one or more of the FACL enzymes. The present invention provides use in the preparation of a medicament of a fatty acid preparation containing a) more than 70% eicosapentaenoic acid or eicosapentaenoic acid derivative and less than 10% docosahexaenoic acid or docosahexaenoic acid derivative and less than 10% linoleic acid or linoleic acid derivative with b) an enzyme inhibitor selected from an inhibitor of COX-1 and/or COX-2, an inhibitor of LOX and an inhibitor of one or more of the FACL enzymes. Such medicaments are to treat any of the following diseases or disorders:
any form of psychiatric disease including schizophrenia, schizoaffective disorders, schizotypy, depression, anxiety, bipolar disorder, mania, borderline personality disorder, alcoholism and attention deficit hyperactivity disorder or any other psychiatric illness; and
any form of neurological or neurodegenerative disease including Parkinson's disease, Alzheimer's disease, Huntington's disease, amyotrophic lateral sclerosis or any other "triplet repeat" disease, multi-infarct or other form of dementia, multiple sclerosis, chronic fatigue and epilepsy.

The main fatty acid or derivative of the fatty acid preparation should have a purity level such that interference at the key points of biological action from other fatty acids or fatty acid derivatives is reduced. The fatty acid or derivative present in the fatty acid preparation used in the present formulations should be at least 70% pure and preferably at least 80% pure. It is especially preferred that the fatty acid or derivative is 90% or 95% pure. In particular, there are two essential fatty acids which are commonly found in oils and which can play a role in the interference of the actions of the therapeutic fatty acids: docosahexaenoic acid and linoleic acid. It is a requirement that the docosahexaenoic acid or derivative and the linoleic acid or derivative present is each less than 10%, preferably less than 5% and very preferably less than 1% of any preparation of a fatty acid or fatty acid derivative used in the formulations of the present invention.

Eicosapentaenoic acid, EPA, is the most important essential fatty acid used in the fatty acid preparation of the present formulations, but it can be replaced by or added to by preparations containing any one or more of gamma-linolenic acid (GLA), dihomogamma-linolenic acid (DGLA), arachidonic acid (AA) and stearidonic acid (SA). In each case, the preparations of these other fatty acids should contain low levels of docosahexaenoic acid and linoleic acid as described in the previous paragraph.

Derivatives of the essential fatty acid which may be used in the present invention include: salts such as sodium, potassium or lithium salts; esters such as ethyl esters and cholesterol esters; mono-, di- and triglycerides; amides; phospolipids; and any other derivatives able to raise the levels of the fatty acid in the blood or tissues.

The enzyme inhibitor is preferably a combined inhibitor of COX-1 and COX-2, or a selective COX-2 inhibitor, or an inhibitor of one of the LOX group of enzymes, or a combined inhibitor of both COX and LOX enzymes. Those of particular interest include inhibitors of 5-lipoxygenase, which inhibit both COX-1 and COX-2, or which inhibit COX-2 selectively. Examples of selective or relatively selective COX-2 inhibitors are celecoxib, rofecoxib parecoxib, valdecoxib, etoricoxib and several other "coxibs", nabumetone, nimesulide, meloxicam, chromene and aroylnapthalene compounds reported in WO 9847890 and WO 9832732, and a range of compounds such as those described in G Dannhardt and S Laufer, Current Medicinal Chemistry 2000; 7: 1101-12. Examples of non-selective or relatively non-selective COX-1 and COX-2 inhibitors include salicylic acid derivatives such as aspirin, sodium salicylate and sulfasalazine, para-aminophenol derivatives such as acetaminophen, indole and indene acetic acids such as indomethacin and sulindac, heteroaryl acetic acids such as tolmetin and diclofenac, arylpropionic acids such as ibuprofen, naproxen and ketoprofen, fenamates such as mefenamic acid and enolic acids such as piroxicam and phenylbutazone.

Work has shown the administration of EFAs to have therapeutically beneficial results in the treatment of many diseases. The advantages of the present invention will be therefore widespread. Case studies follow, but the applications are expected to be diverse, based on the present and future knowledge of the uses of EFAs.

It is surprising that the therapeutic effects of EFAs and derived EFAs may be substantially enhanced by combining administration of the EFA with a drug which blocks the conversion of the EFA to its metabolites. Drugs which block the COX or LOX or FACL groups of enzymes may be of particular interest.

This is an unexpected proposal because it is generally believed that the COX and LOX enzymes inhibitors exert their therapeutic effects not by conserving fatty acids but by blocking their conversion to prostaglandins, leukotrienes and other eicosanoids. It was conventionally thought that the lowering of eicosanoid levels is the critical mechanism of action; and a Nobel Prize was awarded to Vane, Samuelsson and Bergstrom for proposing this, so it is clearly a mainstream concept. The last thing any skilled person would want to do therefore is to propose administering substrates for the COX and LOX enzymes at the same time as providing COX and LOX inhibitors. Such an action would enhance the formation of the eicosanoids whose production the drug was designed to block. Indeed investigators have often proposed that the way to improve therapy in diseases which may respond to COX and LOX inhibitors is to reduce the levels of the relevant fatty acids by dietary or other means. Thus, for example, a major pharmaceutical company which is expert in the field of fatty acids and of prostaglandin synthesis is developing inhibitors of delta-6- and delta-5-desaturases as anti-inflammatory agents (MG Obukowicz et al, J Pharmacol Exp Ther 1998; 287: 157-166). The aim of these drugs is to reduce the levels of prostaglandin precursors such as arachidonic acid, dihomogammalinolenic acid and eicosapentaenoic acid. Since the COX and LOX inhibitors are anti-inflammatory agents also, this teaching points completely away from the idea that the therapeutic effects of COX and LOX inhibitors might actually be enhanced by increasing the levels of these fatty acids.

In contrast, the result of the administration of the formulations used in the present invention is that the therapeutic effects of EFAs and of drugs which inhibit EFA metabolism by LOX, COX, or FACL enzyme will be dramatically enhanced by the co-administration of the fatty acid with the drug. This concept may be applied to any present or future LOX, COX or FACL inhibitors. Drugs of particular interest in this respect are compounds which inhibit COX-1 and COX-2, or COX-2 selectively, or LOX selectively or COX and LOX together. This is because these compounds are widely used and understood and are readily available for administration.

The components of the medicament may be prepared for co-administration, whether in a single or separate formulation of one or more EFA or derived EFAs, selected from GLA, DGLA, AA, SA and EPA, preferably eicosapentaenoic acid and/or gamma-linolenic acid, which are well tolerated in high doses, together with one or more drugs which inhibit COX-1 or COX-2, one or more of the LOX enzymes or one or more of the FACL enzymes. Drugs of particular interest are ones which inhibit 5-lipoxygenase, which inhibit both COX-1 and COX-2, or which inhibit COX-2 selectively. The fatty acids may be used in doses from 5mg to 50g/day, preferably 100mg to 20g/day and very preferably from 500mg to 10g/day. They may be used in any appropriate form which will raise the levels of the fatty acids in bodily tissues. Appropriate forms may include free acids, salts, esters such as ethyl esters mono-, di-, and triglycerides, amides, cholesterol esters, phospholipids, and any other appropriate forms. The enzyme inhibitors may be used in the doses which have been found to be safe and effective for each individual drug. Other conventional pharmaceutical ingredients may be present. The blue-green algae spirulina is not included as a therapeutic agent which may be used in the present formulations because in its native form the oil is likely to contain too much linoleic acid and not enough of any of the target fatty acids. The present formulations are intended for oral administration. Topical application routes are not included.

The aim of such combined administration is to elevate the levels of the fatty acids in cells by providing the fatty acid or its precursor, together with a drug which blocks the metabolism of the fatty acid by one or other of its metabolic routes. The invention may be illustrated by the following examples:

### EXAMPLES

A 45-year-old woman had been seriously depressed for over 10 years and had failed to respond to treatment with several different antidepressants of various classes. Because there is evidence of elevated formation of prostaglandins in depression, she was given a combined COX-1 and COX-2 inhibitor, ibuprofen. She thought this might have had a marginal effect but this was not apparent to observers. The ibuprofen was stopped and she was treated with ethyl-eicosapentaenoate at a dose of 1g/day. This seemed to have a modest effect but she remained far from well. However, when ibuprofen was added to the EPA there was a dramatic improvement and she has remained well for four months. The combination of ibuprofen, a COX-1 and COX-2 inhibitor, with EPA had a substantial beneficial effect which was achieved by neither drug alone.

An 81-year-old woman had become very forgetful and her family was concerned that she might be developing Alzheimer's disease (AD). Because there is evidence that the NSAID indomethacin may be able to slow down the progression of AD, her son, who was a doctor, prescribed indomethacin. Over six months or so this may have had a slight effect but there was no dramatic change. Because of animal work on the beneficial effects of AA in the aging rat brain, 800mg of AA in triglyceride form was added to the treatment regime. Over the following 12 weeks the patient experienced a substantial renewal of energy, became more aware of what was going on around her and showed a considerable improvement in her memory for daily events.

These case histories illustrate the benefits of combining in therapy an EFA together with an inhibitor of COX-1, COX-2 or the LOX enzymes.

### Example Formulations

1. Formulations of hard or soft gelatin capsules in which each capsule contains between 100mg and 1000mg of eicosapentaenoic acid in ethyl ester or triglyceride form, where the EPA preparation contains at least 70% eicosapentaenoic acid derivative, less than 10% docosahexaenoic acid or derivative, and less than 10% linoleic acid or derivative, together with an inhibitor of COX-1 or COX-2 or LOX or a drug which has multiple inhibitory effects on those enzymes. The fatty acid dose should be adjusted to provide between 50mg and 10,000mg daily and the daily dose of fatty acid should also provide for an appropriate daily dose of the COX or LOX inhibitor.
2. Combination packs in which the eicosapentaenoic acid of example formulation 1 is provided in a hard or soft gelatin capsule while the COX or LOX inhibitor is provided in a tablet, capsule or other appropriate dosage form, the two types of dosage form being provided in the same overall pack with a set of instructions for their combined use.
3. Formulations as in 1 and 2 where the eicosapentaenoic acid preparation contains more than 90% eicosapentaenoic acid and where the levels of linoleic acid or of docosahexaenoic acid are each below 5%, and preferably below 1%.
4. As in example formulations 1 to 3 where the EPA is replaced by or supplemented with a fatty acid preparation selected from GLA, DGLA, AA and SA.
5. As in example formulations 1 to 4 where the drug is a non-steroidal anti-inflammatory agent with a dual action on COX-1 and COX-2 such as aspirin, ibuprofen, indomethacin or any one of the many drugs in this class.
6. As in example formulations 1 to 4 where the drug is a selective COX-2 inhibitor such as celecoxib, rofecoxib or any other selective inhibitor.
7. As in example formulations 1 to 4 where the drug is a compound which has LOX inhibitory activity with or without COX inhibitory activity.
8. As in example formulations 1 to 4 where the drug is a compound which inhibits fatty acid coenzyme-A ligases (FACL).
9. As in example formulations 1 to 4 where two or more drugs with COX-, LOX- or FACL-inhibiting activity are combined.

## Claims

1. Use of a fatty acid preparation containing:
a) more than 70% eicosapentaenoic acid (EPA) or eicosapentaenoic acid derivative and less than 10% docosahexaenoic acid or a docosahexaenoic acid derivative and less than 10% linoleic acid or a linoleic acid derivative; and
b) an enzyme inhibitor selected from an inhibitor of COX-1 and/or COX-2, an inhibitor of LOX and an inhibitor of one or more of the FACL enzymes
in the preparation of a medicament for the treatment of any of the following diseases or disorders:
any form of psychiatric disease including schizophrenia, schizoaffective disorders, schizotypy, depression, anxiety, bipolar disorder, mania, borderline personality disorder, alcoholism and attention deficit hyperactivity disorder or any other psychiatric illness; and
any form of neurological or neurodegenerative disease including Parkinson's disease, Alzheimer's disease, Huntington's disease, amyotrophic lateral sclerosis or any other "triplet repeat" disease, multi-infarct or other form of dementia, multiple sclerosis, chronic fatigue and epilepsy.

2. Use according to claim 1, in which the fatty acid preparation contains more than 80% eicosapentaenoic acid or eicosapentaenoic acid derivative and less than 5% docosahexaenoic acid or a docosahexaenoic acid derivative and less than 5% linoleic acid or a linoleic acid derivative.

3. Use according to claim 2, in which the fatty acid preparation contains more than 90% eicosapentaenoic acid or eicosapentaenoic acid derivative and less than 5% docosahexaenoic acid or a docosahexaenoic acid derivative and less than 5% linoleic acid or a linoleic acid derivative.

4. Use according to claim 1 in which the fatty acid preparation contains more than 90% eicosapentaenoic acid or eicosapentaenoic acid derivative and less than 1% docosahexaenoic acid or a docosahexaenoic acid derivative and less than 1% linoleic acid or a linoleic acid derivative.

5. Use according to claim 4 in which the fatty acid preparation contains more than 95% eicosapentaenoic acid or eicosapentaenoic acid derivative and less than 1% docosahexaenoic acid or a docosahexaenoic acid derivative and less than 1% linoleic acid or a linoleic acid derivative.

6. Use according to any of claims 1 to 5 in which the fatty acid preparation is in the form of the free acid and/or a derivative selected from: salts including sodium, potassium or lithium salts; esters such as ethyl esters and cholesterol esters; mono-, di- and triglycerides; amides; phospolipids; and any other derivatives able to raise the levels of the fatty acid in the blood or tissues.

7. Use according to any of claims 1 - 6 in which the EPA is replaced by or added to by any one or more of preparations of gamma-linolenic acid (GLA), dihomogammalinolenic acid (DGLA), arachidonic acid (AA) and stearidonic acid (SA), each containing less than 10% docosahexaenoic acid and less than 10% linoleic acid.

8. Use according to any preceding claim in which the enzyme inhibitor is a combined inhibitor of COX-1 and COX-2, or a selective COX-2 inhibitor, or an inhibitor of one of the LOX groups of enzymes, or a combined inhibitor of both COX and LOX enzymes.

## Patentansprüche

1. Verwendung eines Fettsäurepräparates, das Folgendes enthält:
a) mehr als 70 % Eicosapentaensäure (EPA) oder Eicosapentaensäurederivat und weniger als 10 % Docosahexaensäure oder eines Docosahexaensäurederivats und weniger als 10 % Linolsäure oder eines Linolsäurederivats; und
b) einen Enzyminhibitor, ausgewählt aus einem Inhibitor von COX-1 und/oder COX-2, einem Inhibitor von LOX und einem Inhibitor von einem oder mehreren der FACL-Enzyme,
zur Herstellung eines Medikaments zur Behandlung einer beliebigen der folgenden Krankheiten oder Störungen:
jede beliebige Form einer psychiatrischen Erkrankung, einschließlich Schizophrenie, schizoaffektive Störungen, Schizotypie, Depression, Angst, bipolare Störung, Manie, Borderline-Persönlichkeitsstörung, Alkoholismus und Aufmerksamkeitsdefizit-Hyperaktivitätsstörung oder einer beliebigen anderen psychiatrischen Krankheit; und
jede beliebige Form von neurologischer oder neurodegenerativer Erkrankung, einschließlich Parkinsonsche Krankheit, Alzheimersche Krankheit, Huntingtonsche Krankheit, amyotrophe Lateralsklerose oder eine beliebige andere "Triplet Repeat"-Krankheit, Multiinfarkt oder eine andere Art der Demenz, multiple Sklerose, chronische Müdigkeit und Epilepsie.

2. Verwendung nach Anspruch 1, wobei das Fettsäurepräparat mehr als 80 % Eicosapentaensäure oder Eicosapentaensäurederivat und weniger als 5 % Docosahexaensäure oder eines Docosahexaensäurederivats und weniger als 5 % Linolsäure oder eines Linolsäurederivats enthält.

3. Verwendung nach Anspruch 2, wobei das Fettsäurepräparat mehr als 90 % Eicosapentaensäure oder Eicosapentaensäurederivat und weniger als 5 % Docosahexaensäure oder eines Docosahexaensäurederivats und weniger als 5 % Linolsäure oder eines Linolsäurederivats enthält.

4. Verwendung nach Anspruch 1, wobei das Fettsäurepräparat mehr als 90 % Eicosapentaensäure oder Eicosapentaensäurederivat und weniger als 1 % Docosahexaensäure oder eines Docosahexaensäurederivats und weniger als 1 % Linolsäure oder eines Linolsäurederivats enthält.

5. Verwendung nach Anspruch 4, wobei das Fettsäurepräparat mehr als 95 % Eicosapentaensäure oder Eicosapentaensäurederivat und weniger als 1 % Docosahexaensäure oder eines Docosahexaensäurederivats und weniger als 1 % Linolsäure oder eines Linolsäurederivats enthält.

6. Verwendung nach einem der Ansprüche 1 bis 5, wobei das Fettsäurepräparat in Form der freien Säure und/oder eines Derivats vorliegt, ausgewählt aus: Salzen, einschließlich Natrium-, Kalium- oder Lithiumsalzen; Estern wie Ethylester und Cholesterolester; Mono-, Di- und Triglyceriden; Amiden; Phospholipiden; und beliebigen anderen Derivaten, die die Werte der Fettsäure im Blut oder Gewebe anheben können.

7. Verwendung nach einem der Ansprüche 1 bis 6, wobei die EPA ersetzt oder ergänzt wird durch eines oder mehrere der Präparate aus Gamma-Linolensäure (GLA), Dihomogamma-Linolensäure (DGLA), Arachidonsäure (AA) und Stearidonsäure (SA), die jeweils weniger als 10 % Docosahexaensäure und weniger als 10 % Linolsäure enthalten.

8. Verwendung nach einem der vorherigen Ansprüche, wobei der Enzyminhibitor ein kombinierter Inhibitor von COX-1 und COX-2 oder ein selektiver COX-2-Inhibitor oder ein Inhibitor von einer der LOX-Enzymgruppen oder ein kombinierter Inhibitor von sowohl COX- als auch LOX-Enzymen ist.

## Revendications

1. Utilisation d'une préparation d'acides gras contenant :
a) plus de 70% d'acide éicosapentaénoïque (EPA) ou d'un dérivé de l'acide éicosapentaénoïque et moins de 10% d'acide docosahexaénoïque ou d'un dérivé de l'acide docosahexaénoïque et moins de 10% d'acide linoléique ou d'un dérivé de l'acide linoléique; et
b) un inhibiteur d'enzymes sélectionné parmi un inhibiteur de COX-1 et/ou de COX-2, un inhibiteur de LOX et un inhibiteur d'une ou de plusieurs enzymes FACL dans la préparation d'un médicament pour le traitement de l'une quelconque des maladies ou troubles suivants :
une forme quelconque de maladie psychiatrique englobant la schizophrénie, les troubles schizo-affectifs, la schizotypie, la dépression, l'anxiété, le trouble bipolaire, la manie, le trouble de la personnalité état limite, l'alcoolisme et le déficit de l'attention, le trouble d'hyperactivité ou toute autre maladie psychiatrique quelconque; et
une forme quelconque de maladie neurologique ou neurodégénérative englobant la maladie de Parkinson, la maladie d'Alzheimer, la maladie de Huntington, la sclérose latérale amyotrophique ou toute maladie à triplet répété, les infarctus multiples ou autre forme de démence, la sclérose en plaques, la fatigue chronique et l'épilepsie.

2. Utilisation selon la revendication 1, dans laquelle la préparation d'acides gras contient plus de 80% d'acide éicosapentaénoïque ou d'un dérivé d'acide éicosapentaénoïque et moins de 5% d'acide docosahexaénoïque ou d'un dérivé d'acide docosahexaénoïque et moins de 5% d'acide linoléique ou d'un dérivé d'acide linoléique.

3. Utilisation selon la revendication 2, dans laquelle la préparation d'acides gras contient plus de 90% d'acide éicosapentaénoïque ou d'un dérivé d'acide éicosapentaénoïque et moins de 5% d'acide docosahexaénoïque ou d'un dérivé d'acide docosahexaénoïque et moins de 5% d'acide linoléique ou d'un dérivé d'acide linoléique.

4. Utilisation selon la revendication 1, dans laquelle la préparation d'acides gras contient plus de 90% d'acide éicosapentaénoïque ou d'un dérivé d'acide éicosapentaénoïque et moins de 1 % d'acide docosahexaénoïque ou d'un dérivé d'acide docosahexaénoïque et moins de 1% d'acide linoléique ou d'un dérivé d'acide linoléique.

5. Utilisation selon la revendication 4, dans laquelle la préparation d'acides gras contient plus de 95% d'acide éicosapentaénoïque ou d'un dérivé d'acide éicosapentaénoïque et moins de 1% d'acide docosahexaénoïque ou d'un dérivé d'acide docosahexaénoïque et moins de 1% d'acide linoléique ou d'un dérivé d'acide linoléique.

6. Utilisation selon l'une quelconque des revendications 1 à 5, dans laquelle la préparation d'acides gras est sous forme de l'acide libre et/ou d'un dérivé sélectionné parmi: des sels, y compris des sels de sodium, de potassium ou de lithium; des esters, tels que des esters éthyliques et des esters de cholestérol; des monoglycérides, des diglycérides et des triglycérides; des amides; des phospholipides; et n'importe quels autres dérivés capables d'élever les taux de l'acide gras dans le sang ou des tissus.

7. Utilisation selon l'une quelconque des revendications 1 - 6, dans laquelle l'EPA est remplacé par ou ajouté à l'une quelconque ou plusieurs préparations d'acide gamma-linolénique (GLA), d'acide dihomogamma-linolénique (DGLA), d'acide arachidonique (AA) et d'acide stéaridonique (SA), contenant chacune moins de 10% d'acide docosahexaénoïque et moins de 10% d'acide linoléique.

8. Utilisation selon l'une quelconque des revendications précédentes, dans laquelle l'inhibiteur d'enzymes est un inhibiteur combiné de COX-1 et de COX-2, ou un inhibiteur sélectif de COX-2, ou un inhibiteur de l'un des groupes d'enzymes LOX, ou un inhibiteur combiné des enzymes COX et LOX.
